# EUROPEAN PATENT SPECIFICATION

(11) **EP 4 126 106 B1**
(45) Date of publication and mention of the grant of the patent: **29.04.2026**
(21) Application number: 21716842.6
(22) Date of filing: 30.03.2021
(51) Int. Cl.: A61M 1/36

(54) **APPARATUS FOR EXTRACORPOREAL REMOVAL OF ALBUMIN AND ENDOTOXIN FROM BLOOD**
GERÄT ZUR EXTRAKORPORALEN ENTFERNUNG VON ALBUMIN UND ENDOTOXINEN AUS DEM BLUT
APPAREIL POUR L'ÉLIMINATION EXTRACORPORELLE D'ALBUMINE ET ENDOTOXINES DU SANG

(30) Priority: 31.03.2020 GB 202004775
(43) Date of publication of application: 08.02.2023
(73) Proprietor: Yaqrit Limited, Ledbury HR8 1RZ (GB)
(72) Inventor: JALAN, Rajiv, Ledbury Hertfordshire HR8 1RZ (GB)
(74) Representative: J A Kemp LLP
(86) International application number: PCT/GB2021/050786
(87) International publication number: WO 2021/198674

(56) References cited:
- LEE KARLA C L ET AL: "Extracorporeal liver assist device to exchange albumin and remove endotoxin in acute liver failure: Results of a pivotal pre-clinical study", JOURNAL OF HEPATOLOGY, ELSEVIER, AMSTERDAM, NL, vol. 63, no. 3, 1 May 2015 (2015-05-01), pages 634 - 642, XP029259378, ISSN: 0168-8278, DOI: 10.1016/J.JHEP.2015.04.020
- E.A.S.L.: "ALIVER - an EU funded project to develop a liver dialysis machine revealed at ILC 2017", 21 April 2017 (2017-04-21), XP055758047, Retrieved from the Internet <URL:https://www.eurekalert.org/pub_releases/2017-04/eaft-a-a042117.php> [retrieved on 20201208]

## Description

### Field of the invention

The invention relates to exogenous albumin for use in the treatment of an individual suffering from a cytokine storm syndrome and to an *in vitro* method for treating said syndrome.

### Background of the invention

Overreactions of the body's immune system can be fatal. Such overreactions are termed cytokine storms, and are characteristic of a variety of conditions such as autoimmune conditions, secondary haemophagocytic lymphohistiocytosis (sHLH), severe viral infections, severe sepsis, post-surgical multi organ failure, post-liver resection multi organ failure, and post chemotherapy cytokine storm.

Cytokines are a diverse group of small proteins that are secreted by cells for the purpose of intercellular signaling and communication. Specific cytokines have autocrine, paracrine, and/or endocrine activity and, through receptor binding, can elicit a variety of responses, depending upon the cytokine and the target cell. Among the many functions of cytokines are the control of cell proliferation and differentiation and the regulation of angiogenesis and immune and inflammatory responses

Cytokine storm syndrome (CSS) is a form of systemic inflammatory response syndrome that can be triggered by a variety of factors such as infections and certain drugs. Cytokine storms are associated with a wide variety of infectious and noninfectious diseases. A cytokine storm occurs when large numbers of white blood cells are activated and release inflammatory cytokines, which in turn activate yet more white blood cells. The symptoms of cytokine storm syndrome can include high fever, enlarged spleen, excessive bleeding, low counts of all types of blood cells (red, white and platelets) and, potentially, multiple organ failure.

Cytokine storms caused by influenza infection are often associated with a surge of activated immune cells into the lungs. The resulting lung inflammation and fluid accumulation can lead to respiratory distress and can be contaminated by a secondary bacterial pneumonia, often enhancing the mortality in patients.

Human Coronaviruses (hCoVs) belong to the virus family Coronaviridae and are enveloped, positive-sense RNA viruses. Generally they can be classified into low pathogenic CoVs and highly pathogenic CoVs according to the consequence after infecting human. High pathogenic CoVs include Severe acute respiratory syndrome CoV (SARS-CoV), Middle East respiratory syndrome CoV (MERS-CoV), and the newly emerging severe acute respiratory syndrome coronavirus 2 (SARS-CoV-2) which causes Coronavirus Disease 2019 (COVID-19). High pathogenic CoVs mainly infect the lower respiratory tract and cause fatal pneumonia, sometimes leading to fatal acute lung injury and acute respiratory distress syndrome(ARDS), resulting in high morbidity and mortality (Channappanavar R, Perlman S 2017).

Recent evidence also suggests that a subgroup of patients with severe COVID-19 also have a cytokine storm syndrome. Predictors of fatality from a recent retrospective, multicentre study of 150 confirmed COVID-19 cases in Wuhan, China, included elevated ferritin and IL-6 suggesting that mortality might be due to virally driven hyperinflammation, and that the most common reason for death in COVID-19 patients is respiratory failure from Acute Respiratory Distress Syndrome (ARDS) (Ruan Q et al. 2020). A retrospective study focused on 113 deceased COVID-19 patients showed older age, male sex and multi-organ dysfunction were more frequent in patients that died compared with patients who recovered (Chen T et al., 2020). Leukocytosis, decreased lymphocytes, low albumin and elevated CRP levels were also found to be more frequent in the patients that died (Chen T et al., 2020). Other poor prognosis prediction factors were higher Sequential Organ Failure Assessment (SOFA) score and D-dimer level >1 microgram/L (Zhou F et al., 2020). For the clinical course of critically ill patients, a Chinese retrospective study reported that among 52 critically ill patients, 61.5% died by 28 days, and the median time from admission to ICU to death was 7 days (Yang X et al., 2020). Age, co-morbidities, organ failures, hypoalbuminemia, and marked inflammatory response are independent determinants of mortality of patients with COVID-19.

Public health interventions such as social isolation are aimed at prevention of viral infection. There is intense activity directed at trying to reduce viral replication using a variety of strategies. The therapeutic options for COVID-19 are limited once the patient develops systemic inflammation and starts to experience organ dysfunction. Several methods have been proposed to control the systemic inflammation in COVID-19. Corticosteroids are used generally to suppress inflammatory conditions. However, the use of steroid in COVID-19 remains controversial (Russell CD et al., 2020; Shang L et al., 2020). Observational data from SARS-CoV and MERS-CoV suggest increased mortality and secondary infection rates, impaired clearance and complications of corticosteroid therapy in survivors (Russell CD et al., 2020). No clinical data has been published regarding the efficacy and safety of steroid in COVID-19. Another approach being considered is the use of an IL-6 antibody to reduce the severity of inflammation. However, once the patient requires organ support on the ICU, management is limited to provision of multiple organ support. Yet, despite the best supportive therapy mortality is high: therapies that target the cytokine storm in COVID-19 patients represent an unmet medical need.

There is thus a need to treat individuals with cytokine storm syndromes, or diseases or conditions associated with cytokine storms, in order to reduce the mortality of such individuals.

### Summary of the invention

The invention relates to the treatment of or diseases or conditions associated with cytokine storms, cytokine storm syndromes, or the treatment of patients with severe cytokine storm. The invention addresses crucial factors which may influence morbidity and mortality, namely defects in albumin structure, function and levels in patients with cytokine storm syndromes, and increased levels of endotoxin in the blood of such patients. The invention also addresses increased levels of cytokines in the blood of such patients.

The present invention is based on the discovery of the inventor that patients suffering from cytokine storm syndromes, such as COVID-19 patients, had marked similarities in the inflammatory response and the associated immunopathology to patients with Acute-on-chronic-liver failure (ACLF). Thus, devices that have been shown to address the cytokinemia and organ failures that are characteristic of ACLF (see Lee *et al*, 2015) would address the cytokine storm characteristics of conditions such as severe COVID-19 infection, and reduce the mortality of these patients.

Accordingly, the invention provides albumin that does not derive from the blood of an individual to be treated for use in a method of a treating a cytokine storm syndrome, said method comprising the steps of:
(a) removing albumin from the blood of the individual;
(b) reducing the level of endotoxin in the blood of the individual; and
(c) optionally introducing said albumin that does not derive from the individual to be treated into the blood of the individual;
wherein step (a) is carried out by dialysis using means comprising a membrane having a pore size of greater than 50kDa and less than 100 kDa.

The invention also provides an *in vitro* method of treating blood extracorporeally by selectively removing albumin and endotoxin from the blood, wherein the blood is from an individual having a cytokine storm syndrome, the method comprising:
(a) contacting the blood with a solid support which selectively binds albumin and thereby removing albumin from the blood;
(b) contacting the blood with a solid support which selectively binds endotoxin and thereby removing endotoxin from the blood; and
(c) optionally, adding to the blood albumin that does not derive from the same individual as the blood.

The invention also provides albumin that does not derive from the blood of an individual to be treated for use in a method of treating an individual with a COVID-19 infection, said method comprising treating said individual's blood by:
(a) selectively removing albumin from the blood of the individual;
(b) selectively removing endotoxin from the blood of the individual; and
(c) optionally supplying albumin that does not derive from the individual to the blood of the individual;
wherein said step (a) comprises dialysis of albumin with means comprising a membrane having a pore size of greater than 50 kDa and less than 100 kDa.

The invention also provides albumin that does not derive from the blood of an individual to be treated for use in a method of treating a COVID-19 patient with multiple organ failure, said method comprising treating said individual's blood by:
(a) selectively removing albumin from the blood of the individual;
(c) selectively removing endotoxin from the blood of the individual; and
(c) optionally supplying albumin that does not derive from the individual to the blood of the individual;
wherein said step (a) comprises dialysis of albumin with means comprising a membrane having a pore size of greater than 50 kDa and less than 100 kDa.

### Brief description of the Figures

**Figure 1****: The configuration of DIALIVE, a device that incorporates two filters to target albumin function and cytokine storm that is a feature of Acute-on- chronic liver failure (ACLF).**
**Figure 2****: Study design of a randomised and controlled trial of DIALIVE in patients with ACLF.**
**Figure 3****: Performance indicators for DIALIVE. (A)** A significant reduction in Human Non-Mercapt Albumin **(B)** an increase in Human Mercapt albumin **(C)** improvement in binding efficiency of albumin and **(D)** evidence of albumin loss in those treated with the device. (SOC: Standard of Care). **(E)** A change in endotoxin activity assay, which is the second performance indicator.
**Figure 4****: Effect of Organ Function. (A)** A marked reduction in the CLIF-ACLF score in the DIALIVE treated patients was seen compared with those treated with standard of care. (**B**) ACLF Grade distributions per treatment over time.
**Figure 5****: Effect of DIALIVE on circulating Cytokines.** A marked reduction in IL-6, IL-8 and TNFa and an increase in IL-10 in the patients treated with DIALIVE compared with those treated with standard of care.
**Figure 6****: Effect of DIALIVE on circulating markers of Cell Death.** A marked reduction in the M30 and M65 fragments of Cytokeratin 18 (markers of cell death) in the patients treated with DIALIVE and standard of care.
**Figure 7****: Inflammatory response and cytokine storm in ACLF is similar to** that in **severe COVID-19.**
**Figure 8****: Example general apparatus**

### Detailed description of the invention

It is to be understood that different applications of the disclosed methods and apparatus may be tailored to the specific needs in the art. It is also to be understood that the terminology used herein is for the purpose of describing particular embodiments of the invention only.

Throughout this specification, the word "comprise", or variations such as "comprised" or "comprising", will be understood to imply the inclusion of a stated element, integer or step, or group of elements, integers or steps, but not the exclusion of any other element, integer or step, or group of elements, integers or steps.

In addition as used in this specification and the appended claims, the singular forms "a", "an", and "the" include plural references unless the content clearly dictates otherwise. Thus, for example, reference to "syndrome" includes "syndromes", and the like. The terms "cytokine storm syndrome" and "severe cytokine storm" are used interchangeably.

The present invention is based on the unexpected finding that removal of endotoxin and exchanging albumin has had a profound effect on the severity of cytokinemia and also organ function in patients. Therefore, devices and methods that remove endotoxin and exchange albumin can abrogate cytokine storms, which are characterised by cytokinemia and also organ dysfunction. Such devices and methods abrogate damaging pro-inflammatory cytokines (such as IL6, IL8 and TNFa) while increasing the anti-inflammatory cytokine, IL10.

Thus, the present invention concerns the use of devices such as the DIALIVE apparatus, or modified versions of said apparatus, in order to treat individuals or patients with cytokine storm syndromes, or individuals suffering from diseases or conditions associated with a cytokine storm. In a preferred embodiment, the cytokine storm syndrome arises from a viral infection. In a preferred embodiment, the viral infection is COVID-19. The DIALIVE apparatus is described in WO 2008/050148.

Cytokine storm syndromes encompass conditions that involve unchecked inflammatory responses, such as multiple organ failure or dysfunction, Alzheimer's disease and stroke, intoxication from protein bound substances, cholestatic syndromes and severe iron accumulation. Thus, the methods and apparatus described herein can also be used to treat individuals or patients with multiple organ failure or dysfunction, Alzheimer's disease and stroke, intoxication from protein bound substances, cholestatic syndromes or severe iron accumulation.

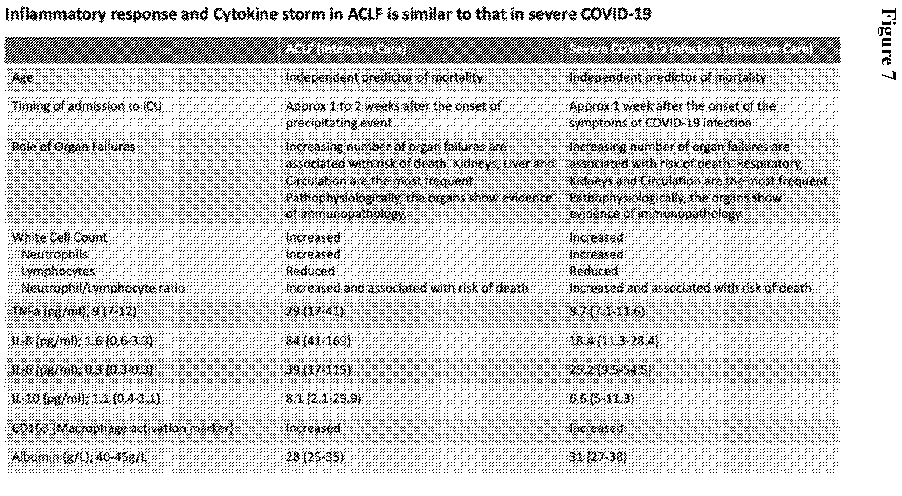

Albumin is the major plasma protein that is produced in the liver. Albumin undertakes a variety of functions including fatty acid transport, metal chelation, drug binding and antioxidant activity. In liver disease such as ACLF its concentration is diminished either due to decreased synthesis or resulting from higher degradation rates. In patients having liver disease such as ACLF, a proportion of the albumin in the blood circulation is structurally abnormal. This albumin profile is consistent with the profile seen in patients with cytokine storm syndromes such as patients with severe COVID-19 (**Figure 7**).

In one aspect, therefore, the invention relates to the removal of albumin from the blood of an individual having a cytokine storm syndrome.

Removal of albumin in this context refers to the removal of structurally normal albumin, and also to the removal of any structurally or functionally modified forms of albumin present in the blood of the individual. That is, preferably, the means for removing albumin used in accordance with the present invention will be capable of removing not only normal, naturally occurring albumin, but also albumin which may have an abnormal structure or albumin which has been modified. Removal of any damaged or abnormal albumin may also be therapeutically useful because damaged albumin has poor functionality and may be associated with damaging side reactions. For example, the means for removing albumin may also remove albumin having reduced molecular flexibility, reduced fatty acid binding affinity, reduced transport quality, reduced transport efficiency and/or reduced detoxification ability compared with normal, unmodified albumin. The means for removing albumin may also remove particular modified forms of albumin, such as ischaemia modified albumin (IMA). Such structural and functional modifications may be detected using conventional techniques. In particular, albumin functionality may be assessed using a spin label and electron paramagnetic resonance spectroscopy. The presence of IMA may be detected by examining the ability of the albumin to bind metal atoms.

Removal of albumin may also detoxify the blood by removing any associated albumin-bound toxins. That is, the means for removing albumin may also consequently remove toxins in the blood that are bound to the albumin.

Preferably, the means for removing albumin from the blood removes albumin selectively. That is, albumin is removed in preference to other substances in the blood, such as other proteins. Preferably the amount of albumin removed from the blood is significantly greater than that of other blood components removed. For example, more than 99% by weight of the component removed in this aspect may be albumin. More than 98%, more than 95%, more than 90%, more than 80%, more than 70%, more than 60% or more than 50% of the component removed in this aspect may be albumin. Removal of albumin here includes the removal of the various modified forms of albumin described herein.

The means for removing albumin may be any means capable of selectively removing albumin from blood.

In one aspect, albumin is selectively removed using a ligand that binds the albumin. The ligand may be any molecule that binds albumin. For example, a number of reactive dyes are known to bind albumin. The ligand may be an antibody or other affinity ligand that specifically binds albumin. Typically, a ligand that specifically binds albumin is a ligand capable of selectively removing albumin from blood as explained above. For example, the ligand may be capable of binding albumin more strongly than other components of blood. For example, the ligand may be an antibody that specifically binds human albumin. The ligand may be an antibody that binds an epitope that is specific to albumin. The ligand may be a combination of molecules which each bind albumin, such as a combination of molecules which bind different parts of the albumin molecule. The ligand may be a polyclonal antibody or mixture of antibodies which bind to multiple epitopes on the albumin protein. Such a combination approach may be useful in the removal of modified forms of albumin as different antibodies may target different parts of the albumin molecule.

Antibodies may be raised against specific epitopes of the albumin molecule. For example, antibodies may be raised specifically against those regions, which are expected to be structurally similar in unmodified and particular modified forms of albumin.

For the purposes of this invention, the term "antibody", unless specified to the contrary, includes fragments which bind albumin. Such fragments include Fv, F(ab') and F(ab')₂ fragments, as well as single chain antibodies. Furthermore, the antibodies and fragment thereof may be chimeric antibodies, CDR-grafted antibodies or humanised antibodies.

Antibodies for use in the present invention can be produced by any suitable method. Means for preparing and characterising antibodies are well known in the art, see for example Harlow and Lane (1988) "Antibodies: A Laboratory Manual", Cold Spring Harbor Laboratory Press, Cold Spring Harbor, NY. For example, an antibody may be produced by raising antibody in a host animal against the whole polypeptide or a fragment thereof, for example an antigenic epitope thereof, herein after the "immunogen".

A method for producing a polyclonal antibody comprises immunising a suitable host animal, for example an experimental animal, with the immunogen and isolating immunoglobulins from the animal's serum. The animal may therefore be inoculated with the immunogen, blood subsequently removed from the animal and the IgG fraction purified.

A method for producing a monoclonal antibody comprises immortalising cells which produce the desired antibody. Hybridoma cells may be produced by fusing spleen cells from an inoculated experimental animal with tumour cells (Kohler and Milstein (1975) Nature 256, 495-497).

An immortalized cell producing the desired antibody may be selected by a conventional procedure. The hybridomas may be grown in culture or injected intraperitoneally for formation of ascites fluid or into the blood stream of an allogenic host or immunocompromised host. Human antibody may be prepared by in vitro immunisation of human lymphocytes, followed by transformation of the lymphocytes with Epstein-Barr virus.

For the production of both monoclonal and polyclonal antibodies, the experimental animal is suitably a goat, rabbit, rat or mouse. If desired, the immunogen may be administered as a conjugate in which the immunogen is coupled, for example via a side chain of one of the amino acid residues, to a suitable carrier. The carrier molecule is typically a physiologically acceptable carrier. The antibody obtained may be isolated and, if desired, purified.

An antibody, or other ligand, "specifically binds" to a protein when it binds with preferential or high affinity to the protein for which it is specific but does substantially bind not bind or binds with only low affinity to other proteins. That is, an antibody specifically binds albumin if it binds to albumin more strongly than it binds to other blood components, such as other proteins in the blood. As explained above, the specificity of binding may be such that it binds structurally or functionally altered forms of albumin as well as unmodified albumin. Preferably it binds structurally or functionally altered forms of albumin with the same or substantially the same binding affinity as unmodified albumin. Preferably, it binds both modified and unmodified forms of albumin with a greater affinity than other blood components, such as other proteins in the blood. A variety of protocols for competitive binding or immunoradiometric assays to determine the specific binding capability of an antibody are well known in the art (see for example Maddox et al, J. Exp. Med. 158, 1211-1226, 1993). Such immunoassays typically involve the formation of complexes between the specific protein and its antibody and the measurement of complex formation.

Where a ligand is used to achieve removal of albumin, the ligand may be provided attached to a solid support. The ligand may be immobilised on such a solid support. A suitable solid support may be in the form of a column through which the blood may be passed. A suitable solid support may be, for example, a porous material such as a membrane, particle bed or filter which is sufficiently porous to allow blood cells to pass through it. A suitable solid support may alternatively be a solid substrate across the surface of which blood may be passed. Preferably the solid support has a large surface area to maximise the area of contact between the blood of the individual and the ligand attached to the support. The solid support may be in the form of beads, which can be filled into a container into which the blood can be inserted, or through which the blood can be passed. The beads will preferably have a size sufficient to allow sufficient porosity when packed into a column or filter bed. Various bead materials are known in the art.

Accordingly, the means for removing albumin according to the present invention may comprise or consist of such a solid support on which is attached or immobilised a ligand capable of binding albumin. The means for removing albumin may comprise or consist of a container through which blood is passed. The container may thus comprise an inlet and an outlet. The inlet and outlet are positioned so that blood passing through the container comes into contact with a solid support as described herein. Preferably the means for removing albumin is designed or selected to maximise the area of contact between the blood and the solid support. A variety of such designs are known in the art. For example, the means for removing albumin may be a column or filter bed packed with beads, wherein a ligand for albumin is immobilised on the beads.

In another aspect, removal of albumin may be achieved via dialysis. Such an approach may also lead to the removal of other blood components that are dialysed together with the albumin. Typically, albumin should be the predominant component removed. This dialysis step can use any albumin dialysis system. A variety of such systems are known in the art. One such system is the molecular absorbance recirculating system (MARS). An alternate is a generic single pass albumin dialysis (SPAD) system. These systems use a 50kDa pore membrane to dialyse albumin in blood. This system is designed particularly to remove albumin-bound toxins from the blood of patients.

As an alternative to this system, a larger pore membrane can be utilised so that albumin from the patient's blood is actively exchanged with fresh albumin via dialysis. This permits the removal of toxins and abnormal forms of albumin in the same step. This also allows the introduction into the blood of new albumin, not from the individual, as discussed further below.

Studies in which the MARS system is modified to include a larger pore membrane indicate that there is a substantial improvement in protein-bound toxin removal compared to the standard 50kDa pore device. For example, a membrane having a pore size of greater than 50 kDa, greater than 60 kDa, greater than 70 kDa, greater than 80 kDa, greater than 90 kDa or greater than 100 kDa may be used. The membrane may have a pore size of less than 60 kDa, less than 75 kDa, less than 100 kDa or less than 150 kDa.

In a preferred embodiment, the membrane has a pore size of greater than 50 kDa and less than 120 kDa. In a preferred embodiment, the membrane has a pore size of at least 50 kDa and less than 120 kDa. In a preferred embodiment, the membrane has a pore size of at least 50 kDa and less than 100 kDa. In a preferred embodiment, the membrane has a pore size of greater than 50 kDa and less than 100 kDa.

In a preferred embodiment the membrane has a pore diameter defined as 10 nm +/two standard deviations.

Removal of albumin can also be a surrogate for the direct removal of cytokines. Thus, in an embodiment of the invention, the removal of albumin results in the removal of cytokines form the blood of the individual.

Other blood components may be removed together with albumin depending on the particular means used to remove the albumin. In one embodiment, other components which are removed with the albumin may be returned to the blood of the individual. The components to be returned may be purified from the albumin mixture that has been removed from the blood, or may be replaced by fresh equivalent components not deriving from the individual.

Various methods of removing albumin from the blood are known in the art. For example, US Patent No. 4,093,612 discloses reactive dye compositions that can be used to remove albumin from a fluid. In accordance with the present invention, such compositions may be used for the removal of albumin from the blood of the individual. This may therefore be a selective albumin trapping system based on compounds which specifically bind albumin. These may be, for example reactive dyes as described in US Patent No. 4,093,612 such as cibacron-blue, or may be other molecules capable of binding albumin, such as albumin-specific antibodies.

In a preferred embodiment, the removal of albumin is carried out using a septeX filter (Gambro) or similar filter.

In accordance with the present invention therefore, albumin is removed from the blood of an individual with liver disease. In one aspect, this albumin may be replaced with new albumin which does not derive from the individual. The new albumin is preferably structurally and functionally normal. That is, the new albumin may comprise no, or substantially no, structurally or functionally modified forms of albumin. Where albumin removed from the blood of an individual comprises one or more modified forms of albumin, the albumin returned to the blood of that individual preferably comprises less modified albumin than has been removed. For example, the albumin returned to the blood of the individual may comprise less than 50%, less than 30%, less than 20%, less than 10%, less than 5% or less than 1% of the amount of modified albumin removed from that individual. Preferably, the albumin returned to the individual will comprise no modified albumin or no modified albumin of one or more of the types that were removed from the individual, such as no ischaemia modified albumin (IMA).

The new albumin may be human albumin. The new albumin may derive from another individual, such as an individual that does not have liver disease, an individual that does not have liver failure or an individual that has normal liver function. The new albumin may be albumin that has been removed from the individual, but has been cleaned or purified to remove toxins and/or modified albumin molecules. The new albumin will typically have a higher proportion of structurally and functionally normal albumin than the albumin removed from the individual. The new albumin can be pharmaceutical grade albumin. The new albumin may be manufactured artificially.

This new albumin is introduced into the blood of the individual to ensure that the individual has a suitable level of circulating albumin. This can be a direct replacement for the albumin removed, for example, the same or an equivalent amount of albumin to that that is removed can be returned to the blood. In this system, the individual's albumin is effectively exchanged with new albumin. Alternatively, by varying the amount of albumin introduced at this stage, the overall albumin concentration in the blood can be increased or decreased if necessary. The amount of albumin that is introduced to the blood may thus be greater than or less than the amount that is removed. For example, cytokine storm syndromes can lead to a decrease in the level of circulating albumin. This can result in a low functional capacity. In accordance with the present invention the amount of new albumin introduced to the blood of the patient may be greater than the amount of albumin removed. This may supplement the level of albumin in the circulation of the individual. For example, an amount of new albumin may be introduced which raises the overall albumin level in the blood to a level the same as, or similar to, that seen in an individual not having liver disease.

The fresh albumin may be introduced to the blood of the individual simultaneously with the removal of the individual's albumin. For example, an exchange of albumin may be achieved by dialysis. Alternatively, the steps of albumin removal and albumin return may be carried out sequentially or separately. For example, where the invention is carried out ex vivo, the blood of the individual may be passed through means for removing albumin and then subsequently have fresh albumin added to it. This may be achieved by different parts of the same apparatus. Alternatively the addition of fresh albumin may be carried out separately. Typically the addition of fresh albumin is carried out after the removal of albumin from the patient's blood.

It has been shown that endotoxin is a component in the blood of individuals with ACLF which may be associated with the prognosis of those patients, for example their susceptibility to infection or organ failure, their risk of mortality and their potential responsiveness to some therapies such as immunosuppression. It has been shown that these prognosis factors are linked to activation of neutrophils in the blood of an individual having ACLF, and that such activation may be related to the presence of a transmissible factor in the plasma of those individuals. Thus, plasma from an individual having a high degree of neutrophil activation is capable of increasing the level of activation of normal neutrophils.

A similar effect may be achieved by contacting normal neutrophils with endotoxin, and that removal of endotoxin from the blood of patients having a high degree of neutrophil activation can reduce the activation levels of neutrophils in that blood. Removal of endotoxin is therefore believed to be useful in the treatment of patients having ACLF whose neutrophils are in an activated state. By restoring normal neutrophil function, the ability of those individuals to combat infection may be improved. The inventor has discovered that the immune profile of patients with ACLF and those with cytokine storm syndromes, such as COVID-19, are markedly similar. Thus, the removal of endotoxins from the blood of patients with cytokine storm syndromes should have the similar benefits to those seen with ACLF patients.

Accordingly, the present invention also relates to the removal of endotoxin from the blood of patients with cytokine storm syndromes. Removal of albumin addresses one issue of detoxification, removal of endotoxin relates to a further issue of reduced immune response. By combining these two approaches in a single apparatus or method, a particularly effective treatment of cytokine storm syndromes is achieved.

Preferably, the means for removing endotoxin from the blood removes endotoxin selectively. That is, endotoxin is removed in preference to other substances in the blood. Preferably the amount of endotoxin removed from the blood is significantly greater than that of other blood components removed. For example, more than 99% by weight of the component removed in this aspect may be endotoxin. More than 98%, more than 95%, more than 90%, more than 80%, more than 70%, more than 60% or more than 50% of the component removed in this aspect may be endotoxin.

The means for removing endotoxin may be any means capable of selectively removing endotoxin from blood.

In one aspect, endotoxin may be selectively removed using a ligand that binds the endotoxin. The ligand may be any molecule that binds endotoxin. For example, anti-endotoxin antibodies, LPS binding proteins, Polymyxin B, polyethyleneimine, an arginine ligand and various peptides are known to bind endotoxin. The ligand may be an antibody or other affinity ligand that specifically bind endotoxin. For example, the ligand may be an antibody that specifically binds endotoxin. Typically, a ligand that specifically binds endotoxin is a ligand capable of selectively removing endotoxin from blood as explained above. For example, the ligand is capable of binding endotoxin more strongly than other components of blood. The ligand may be an antibody that binds an epitope that is specific to endotoxin. The ligand may be a combination of molecules which each bind endotoxin, such as a combination of molecules which bind different parts of the endotoxin molecule or different endotoxins. The ligand may be a polyclonal antibody or mixture of antibodies which bind to multiple epitopes on the endotoxin molecule or different endotoxins.

Antibodies may be raised against specific epitopes of the endotoxin molecule. Suitable antibody types may be any antibody type, as described above in relation to albumin, such as an antibody fragment.

Antibodies that bind endotoxin may be prepared by any means, for example as described above in relation to albumin-binding antibodies. The antibody obtained may be isolated and, if desired, purified.

An antibody, or other ligand, "specifically binds" to a protein when it binds with preferential or high affinity to the protein for which it is specific but does substantially bind not bind or binds with only low affinity to other proteins. That is, it binds to endotoxin more strongly than it binds to other blood components, such as other proteins in the blood. The specificity of binding may be such that it binds different forms of endotoxin. Preferably, it binds a variety of forms of endotoxin with a greater affinity than other blood components.

Where a ligand is used to achieve removal of endotoxin, the ligand may be provided attached to a solid support. The ligand may be immobilised on such a solid support. Suitable solid supports are as discussed above in relation to albumin-binding ligands.

Accordingly, the means for removing endotoxin according to the present invention may comprise or consist of such a solid support on which is attached or immobilised a ligand capable of binding endotoxin. The means for removing endotoxin may comprise or consist of a container through which blood is passed. The container may thus comprise an inlet and an outlet. The inlet and outlet are positioned so that blood passing through the container comes into contact with a solid support as described herein. Preferably the means is designed or selected to maximise the area of contact between the blood and the solid support. A variety of such designs are known in the art. For example, the means may be a column or filter bed packed with beads, wherein a ligand for albumin is immobilised on the beads.

In a preferred embodiment, the removal of endotoxin is carried out using a oXiris filter (Baxter).

In another aspect, rather than physically removing the endotoxin from the blood, an agent may be administered to the individual to reduce endotoxin levels. For example the endotoxin in the blood may be functionally neutralised rather than removed. Various methods for neutralising endotoxin are known in the art. This may comprise administering an agent to the individual, which agent is capable of selectively removing or neutralising the activity of the endotoxin. This may rely on the host immune system to aid removal of endotoxin. For example, a suitable agent may bind endotoxin and allow the immune system of the individual to clear the endotoxin-agent complexes from the blood. Various agents for decreasing circulating endotoxin levels are known, for example anti-endotoxin antibodies, albumin and LPS-binding proteins, LPS neutralising CD-14 antibodies.

Other blood components may be removed together with endotoxin depending on the particular means used to remove the endotoxin. For example, some methods for removing endotoxin may also remove other toxins from the blood. This may be beneficial to the patient. Some methods for removing endotoxin may also remove other blood components which it is desired to maintain in the blood. In this case, blood components which are removed with the endotoxin may be returned to the blood of the individual. The components to be returned may be purified from the endotoxin mixture removed, or may be replaced by fresh equivalent components that do not derive from the individual.

Various approaches for removing endotoxin from a sample have been described in the art. For example, EP-A-0 129 786 describes the use of Polymyxin B covalently immobilized on polystyrene fibres for the removal of endotoxins from blood. Falkenhagen et al (Artificial Organs (1996) 20:420) described the removal of endotoxin from plasma using polyethyleneimine coated beads. WO 01/23413 describes oligopeptides having a high degree of dispersity which are used to selectively remove endotoxin from blood or plasma. US 5,476,715 describes materials for the removal of endotoxin from a sample, which comprise a porous carrier made from polymers of acrylic acid and methacrylic acid with a particular particle size and spacing. Staubach et al (Transfusion and Apheresis Science (2003) 29: 93-98) describes a device for endotoxin adsorption which is based on immobilized albumin. There are thus a number of available methods which could be used to remove endotoxin from a sample. Any of these methods may be used or adapted for use in accordance with the present invention. The skilled reader would be able to select a suitable method and conditions for its use.

The apparatus or method herein described will preferably be effective in achieving a significant reduction in circulating blood endotoxin levels. For example, the apparatus or method may lead to a reduction by at least 25%, at least 50%, at least 70%, at least 80%, at least 90%, at least 95%, at least 99% or more in the level of endotoxin in the blood of the individual.

Albumin is capable of binding endotoxin in the blood. Thus, the removal of albumin may also lead to the removal of some endotoxin which is bound to albumin. Furthermore, by increasing the level of normal albumin in the individual, again, albumin in the blood can bind to circulating endotoxin and the levels of free endotoxin in the blood may be decreased. However, the amount of endotoxin reduction achieved in this way is relatively small, so the present invention preferably utilises separate means for (a) removing albumin and (b) reducing endotoxin levels. This effect of albumin removal and/or replacement may supplement other means for reducing endotoxin and may help to "mop up" endotoxin remaining in the blood of the individual.

Based on these findings, the inventor has developed a new method and apparatus for use in the treatment of individuals having cytokine storm syndromes. These comprise a combination of components which act to remove both albumin and endotoxin from the blood of an individual having liver disease.

Particularly, the inventor has developed an apparatus for use in the treatment of an individual having a cytokine storm syndrome, the apparatus comprising means for selectively removing albumin from the blood of the individual, comprises means for dialysis of albumin comprising a membrane having a pore size of greater than 50 kDa and less than 100 kDa, and means for selectively removing endotoxin from the blood of the individual. Preferably these are two separate means, each directed to achieving one of these effects. The apparatus may also comprise means for supplying new albumin, that does not derive from the individual, to the blood of the individual.

In addition, the apparatus may also comprise means for removing cytokines from the blood of the individual. The means for removing cytokines from the blood may be the same, or different to, the means from removing albumin, and the means for removing endotoxin from the blood of the individual.

Use of such an apparatus thus results in the removal of albumin from the blood of the individual, a reduction in the level of endotoxin in the blood of the individual and optionally the introduction of new albumin to the blood of the individual. This is therapeutically useful in a variety of ways. Removal of endogenous albumin from the blood of an individual may result in the removal of unwanted toxins bound to the albumin and may also result in the removal of abnormal modified forms of albumin from the blood. Such modified forms often have reduced functional ability. The optional introduction of new albumin can be used to replace the albumin that has been removed with fresh albumin. Preferably the fresh albumin does not comprise toxins bound to the albumin molecules and preferably the fresh albumin is in unmodified form. The addition of fresh albumin to the blood can also be used to supplement endogenous albumin levels where these are reduced as a result of the cytokine storm syndrome. Finally, the removal of endotoxin from the blood of an individual having the cytokine storm syndrome can help to reduce the level of activation of neutrophils in the blood of the individual. This reduction in neutrophil activation can lead to decreased risk of infection, organ failure and mortality, and can improve the responsiveness of the individual to immunosuppressive therapy and steroid or antibiotic treatment.

The invention thus provides a targeted approach to the treatment of cytokine storm syndromes, addressing multiple factors linked to such disease and benefiting the individual in multiple ways.

Herein described is an apparatus for use in the treatment of an individual with cytokine storm syndromes. The apparatus may comprise a number of components, which may be used in combination or separately. An apparatus of the invention will comprise or consist essentially of means for selectively removing albumin from the blood of an individual and means for selectively removing endotoxin from the blood of the individual. The apparatus of the invention may comprise means for removing cytokines from the blood of the individual.

Any means described herein may be used for the removal of albumin, endotoxin or cytokines. In one aspect, a single component of the apparatus may be used for the removal of both albumin and endotoxin. Typically, such a component of the apparatus will comprise both means for removing albumin and means for removing endotoxin. For example, where albumin and endotoxin are both to be removed by binding to specific ligands, then one or more ligands specific for albumin and one or more ligands specific for endotoxin may be used together in the same component of the apparatus. For example the apparatus may comprise a single container, such as a column or filter bed, which comprises solid support(s) onto which the two ligands are immobilised. The ligands may be immobilised onto different supports or onto the same support. The albumin and endotoxin may thus be removed simultaneously from the blood of the individual.

Alternatively, the apparatus may include separate means for removal of albumin and endotoxin. The apparatus may include more than one means capable of removing albumin and/or more than one means capable of removing albumin. In one aspect, a single component of the apparatus may be used for the removal of both albumin and cytokines. In one aspect, a single component of the apparatus may be used for the removal of both endotoxin and cytokines. In one aspect, a single component of the apparatus may be used for the removal of both albumin and cytokines and another component of the apparatus may be used for the removal of both endotoxin and cytokines. For example, the septeX filter may remove cytokines in addition to albumin. The oXiris filter may remove cytokines in addition to endotoxin.

The apparatus may comprise further components. For example, the apparatus may comprise means for removing cytokines from the blood of the individual. The apparatus may comprise means for supplying new albumin, i.e. albumin that does not derive from the individual, to the blood. The apparatus may be for use ex vivo or in vitro. For example, the apparatus may be designed such that blood from the individual passes through it so as to achieve albumin removal and endotoxin removal before returning to the body of the individual.

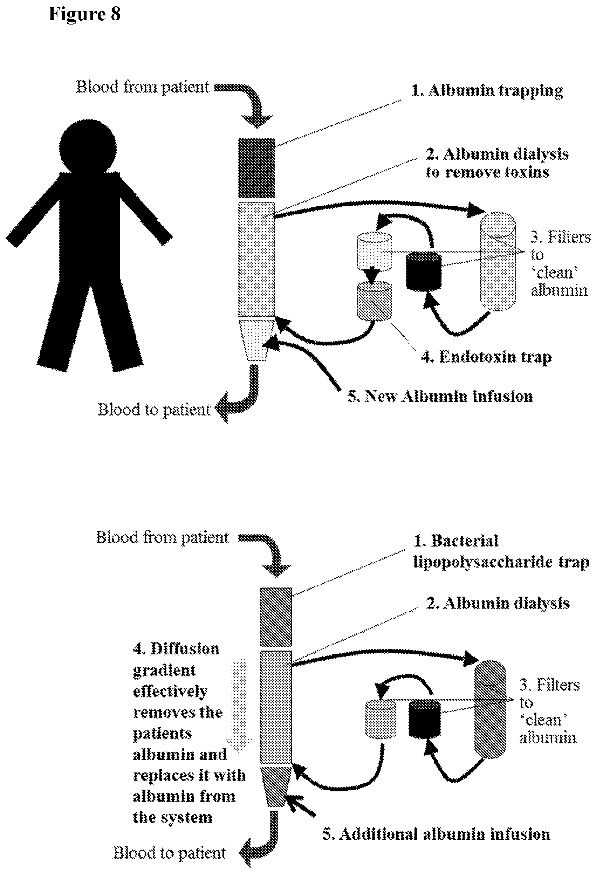

Two possible apparatuses are illustrated in Figure 8 (taken from WO 2008/050148). It will be clear from the discussion herein and the two apparatuses that are exemplified in Figure 8 that a number of different components may be used in a variety of combinations in order to achieve the desired effects. For example, the apparatus of the invention may also remove cytokines.

The components of the first illustrated apparatus in Figure 8 are as follows:
1. Means for trapping albumin. The albumin trap removes albumin from the blood of the patient.
2. Means for carrying out albumin dialysis to remove toxins. For example, the use of a large pore membrane (greater than 50 kDa) to allow albumin exchange permits improved toxin removal by allowing patient albumin to interact with the "cleaning" filters. This also permits the removal of patient albumin as it is exchanged across the filter with the albumin dialysate.
3. Filters to clean albumin.
4. Endotoxin removal component.
5. New albumin infusion.

The components of the second illustrated apparatus in Figure 8 are as follows:
1. Bacterial lipopolysaccharide trap. This is a means for removal of LPS/endotoxin from the blood of the patient.
2. Means for carrying out albumin dialysis to remove toxins. For example as discussed for the first illustrated apparatus in Figure 8.
3. Filters to clean albumin.
4. Albumin replacement means. For example, a diffusion gradient for removing the patients albumin and replacing it with fresh albumin.
5. New albumin infusion.

Herein described is also a method of treating a disease or condition associated with a cytokine storm by using the apparatus of the invention. For example, blood from the individual may be contacted with an apparatus of the invention such that albumin and endotoxin are removed from the blood and albumin that does not derive from the individual may optionally be supplied to the blood. In a preferred embodiment, cytokines are also removed from the blood of the individual. This method may be carried out ex vivo or in vitro and the blood may be subsequently returned to the individual.

Herein described is also a method of treating an individual having a disease or condition associated with a cytokine storm. The method comprises or consists essentially of the following steps: (a) removing albumin from the blood of the individual; and (b) reducing the level of endotoxin in the blood of the individual. This may be achieved by any method or means as described herein. This method may be carried out ex vivo. Step (a) may be achieved using dialysis. Step (a) may be achieved using a ligand capable of specifically binding albumin. Step (b) may be achieved by directly removing endotoxin from the blood. Step (b) may be achieved using a ligand that specifically binds endotoxin. Step (b) may be achieved by administering to the individual a therapeutically effective amount of an agent capable of reducing the level of endotoxin in the blood. Any combination of step (a) and step (b) methods described herein may be used. Optionally the method further includes the step of introducing albumin that does not derive from the individual into the blood of the individual. Optionally the method further includes the step of removing cytokines from the blood of the individual.

Herein described is also a method of treating blood extracorporeally by selectively removing albumin and endotoxin from the blood, wherein the blood is from an individual having a disease or condition associated with a cytokine storm. This method may be achieved by any suitable means as described herein and may comprise the additional step of adding to the blood albumin that does not derive from the individual. Blood which has been treated in this way may be returned to the individual for therapeutic purposes, or may be used for another purpose. For example, blood may be treated in this way prior to transfusion into a different individual.

The methods and apparatus described herein can be used to treat cytokine storm syndromes. Cytokine storm syndromes, or cytokine storms, can be characteristic of, or associated with, disease or conditions such as autoimmune conditions, secondary haemophagocytic lymphohistiocytosis (sHLH), viral infections or severe viral infections, severe sepsis, post-surgical multi organ failure, post-liver resection multi organ failure, and post chemotherapy cytokine storm. In a preferred embodiment, the cytokine storm syndrome to be treated is a viral infection. In a preferred embodiment, the viral infection is influenza or COVD-19.

Cytokine storm syndromes encompass conditions that involve unchecked inflammatory responses, such as multiple organ failure or dysfunction, Alzheimer's disease and stroke, intoxication from protein bound substances, cholestatic syndromes and severe iron accumulation. Thus, the methods and apparatus described herein can also be used to treat individuals or patients with multiple organ dysfunction, Alzheimer's disease and stroke, intoxication from protein bound substances, cholestatic syndromes or severe iron accumulation. In a preferred embodiment, the cytokine storm syndrome to be treated is multiple organ failure or dysfunction.

Multiple organ failure or dysfunction can be defined as the failure of at least two organ systems arising from a physiologic insult such that homeostasis cannot be maintained without medical intervention. Organ systems commonly affected include the central nervous system, the respiratory system, the renal or urinary system, the cardiovascular system, the coagulation system, the gastrointestinal system, the musculoskeletal system and the hepatobiliary system. Multiple organ failure as defined herein can be considered to comprise organ system failure of at least the central nervous system and the respiratory system, or the central nervous system and the renal or urinary system. Multiple organ failure as defined herein can be considered to comprise organ failure of at least the brain and lungs, or the brain and kidney.

Where the level of endotoxin is reduced using an agent to be administered to the individual, the agent may be administered in a variety of dosage forms. Thus, an agent may be administered orally, for example as tablets, troches, lozenges, aqueous or oily suspensions, dispersible powders or granules. The agent may also be administered parenterally, either subcutaneously, intravenously, intramuscularly, intrasternally, transdermally or by infusion techniques. The agent may also be administered in the form of a suppository. A physician will be able to determine the required route of administration for each particular patient.

The formulation of an agent will depend upon factors such as the nature of the exact agent, whether a pharmaceutical or veterinary use is intended, etc. An agent which is to be used to treat liver disease may be formulated for simultaneous, separate or sequential use.

An agent is typically formulated for administration in the present invention with a pharmaceutically acceptable carrier or diluent. The pharmaceutical carrier or diluent may be, for example, an isotonic solution. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, gum arabic, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non-toxic and pharmacologically inactive substances used in pharmaceutical formulations. Such pharmaceutical preparations may be manufactured in known manner, for example, by means of mixing, granulating, tabletting, sugar-coating, or film-coating processes.

Liquid dispersions for oral administration may be syrups, emulsions or suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspensions or solutions for intramuscular injections may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for intravenous administration or infusion may contain as carrier, for example, sterile water or preferably they may be in the form of sterile, aqueous, isotonic saline solutions.

The dose of an agent may be determined according to various parameters, especially according to the substance used; the age, weight and condition of the patient to be treated; the route of administration; and the required regimen.

Again, a physician will be able to determine the required route of administration and dosage for any particular patient. A typical daily dose is from about 0.1 to 50 mg per kg of body weight, according to the activity of the specific inhibitor, the age, weight and conditions of the individual to be treated, the type and severity of the degeneration and the frequency and route of administration. Preferably, daily dosage levels are from 5 mg to 2 g.

### Examples

### Example 1: Repurposing DIALIVE, an extracorporeal liver assist device in order to target severe cytokine storm for the treatment of severe COVID-19 infection (TREAT-COV)

DIALIVE is a novel 'liver dialysis device', developed and optimized by the ALIVER Consortium. The DIALIVE device is based upon the discovery that (i) albumin, a circulating protein involved in detoxification is reduced irreversibly in function and (ii) endotoxemia contributes to increased risk of infection in liver failure. DIALIVE incorporates albumin removal and replacement and, endotoxin removal and is a TRL5. In animal models of liver failure, DIALIVE was shown to be easy to use, safe, reduced endotoxemia and, improved albumin and immune function and, prolonged survival.

Human Coronaviruses (hCoVs) belong to the virus family Coronaviridae and are enveloped, positive-sense RNA viruses. Generally they can be classified into low pathogenic CoVs and highly pathogenic CoVs according to the consequence after infecting human. High pathogenic CoVs include Severe acute respiratory syndrome CoV (SARS-CoV), Middle East respiratory syndrome CoV (MERS-CoV), and the newly emerging severe acute respiratory syndrome coronavirus 2 (SARS-CoV-2) which causes Coronavirus Disease 2019 (COVID-19). High pathogenic CoVs mainly infect the lower respiratory tract and cause fatal pneumonia, sometimes leading to fatal acute lung injury and acute respiratory distress syndrome(ARDS), resulting in high morbidity and mortality (Channappanavar R, Perlman S 2017). Although the emergence of COVID-19 only started several months ago, COVID-19 has already caused more deaths than the total of SARS and MERS (Mahase E 2020). According to World Health Organization latest report (27 March 2020), overall fatality rate is about 4.5% globally. However, the fatality rates vary between different countries such as: Italy - 10%, Iran - 8%, Spain - 7%, UK - 5%, US - 1%, Germany-0.6%, Australia - 0.4% (Coronavirus disease 2019 (COVID-19)-Situation Report). As for China, the fatality rate was estimated to be 2.3% according to a recent 72,314 case-series (The epidemiological characteristics of an outbreak of 2019 novel coronavirus diseases (COVID-19) in China, 2020). Results from limited reports of case series showed that age, pre-existing comorbidities related to the poor outcome of COVID-19.

Age and co-morbidity are the most important independent prognostic factors of mortality. Most deaths in China is among patients aged more than 60 years and/or those who have comorbidities (10.5% for cardiovascular disease, 7.3% for diabetes, 6.3% for chronic respiratory disease, 6% for hypertension, and 5.6% for cancer) (The epidemiological characteristics of an outbreak of 2019 novel coronavirus diseases (COVID-19) in China, 2020). For the US, the fatality rates also vary among different age groups: highest for aged ≥85 years (10% to 27%), then aged 65 to 84 years (3% to 11%), 55 to 64 years (1% to 3%), 20 to 54 years (<1%), and ≤19 years (no deaths) (Severe Outcomes Among Patients with Coronavirus Disease 2019 (COVID-19) - United States, February 12-March 16, 2020). The most common reason for death in COVID-19 patients is respiratory failure from ARDS (Ruan Q et al., 2020). A retrospective study focused on 113 deceased COVID-19 patients showed older age, male sex and multi-organ dysfunction were more frequent in patients that died compared with patients who recovered (Chen T et al., 2020). Leukocytosis, decreased lymphocytes, low albumin and elevated CRP levels were also found to be more frequent in the patients that died (Chen T et al., 2020). Other poor prognosis prediction factors were higher Sequential Organ Failure Assessment (SOFA) score and D-dimer level >1 microgram/L (Zhou F et al, 2020). For the clinical course of critically ill patients, a Chinese retrospective study reported that among 52 critically ill patients, 61.5% died by 28 days, and the median time from admission to ICU to death was 7 days (Yang X et al., 2020). In summary, age, co-morbidities, organ failures, hypoalbuminemia, and marked inflammatory response are independent determinants of mortality. Strategies that impact on organ failure, particularly respiratory, may alter the mortality of COVID-19 patients admitted to the ICU.

Disease progression is often associated with rapid virus replication, massive inflammatory cell infiltration and elevated pro-inflammatory cytokine/chemokine responses. Previous researches on SARS-CoV and MERS-CoV showed that hCoV infection of airway and/or alveolar epithelial cells, rapid virus replication, delayed IFN responses and the monocyte-macrophages and neutrophil accumulation could all contribute to the exacerbated cytokine storm and immunopathology. The excessive inflammatory response can consequently lead to epithelial and endothelial cell apoptosis and vascular leakage, suboptimal T cell response, accumulation of alternatively activated macrophages and altered tissue homeostasis and acute respiratory distress syndrome (Channappanavar R, Perlman S 2017). The systemic inflammation seems to be associated with the severity of hCoVs infection. For instance, high serum levels of pro-inflammatory cytokines (IFN-γ, IL-1, IL-6, IL-12, and TGFβ) and chemokines (CCL2, CXCL10, CXCL9, and IL-8) and low levels of the anti-inflammatory cytokine(IL-10) were found in SARS-CoV or MERS-CoV patients with severe disease compared to individuals with uncomplicated virus infection (Channappanavar R, Perlman S 2017). Similar systemic inflammation is also found in COVID-19 patients. Severe COVID-19 cases present higher neutrophil-lymphocyte-ratio (NLR), higher tumor necrosis factor-α, IL-2R, IL-6, IL-8, IL-10 (Qin C et al., 2020). It has been hypothesized that SARS-CoV-2 can bind to the Toll Like Receptor (TLR) and cause the activation of inflammasome and the release of pro-IL-1β, which is a mediator of lung inflammation, fever and fibrosis (Conti P et al., 2020). Therapies targeting the severity of cytokinemia is a potential therapeutic for patients with COVID-19 infection. Novel therapeutic strategies that are able to reduce the cytokine storm are likely to reduce organ immunopathology and may improve the survival of COVID-19 patients.

### Rationale for repurposing DIALIVE, a liver assist device targeting inflammation and immunopathology in Liver Failure for the treatment of critically-ill COVID-19 patients

Acute on chronic liver failure (ACLF) is a newly defined syndrome that occurs in patients with cirrhosis who deteriorate acutely following due to precipitant events such as infection. Patients manifest with organ failures and depending upon the severity of organ failures, between 20 and 90% of these patients will die. From the pathophysiological perspective, the patients present with a marked systemic inflammation and a massive cytokine storm.

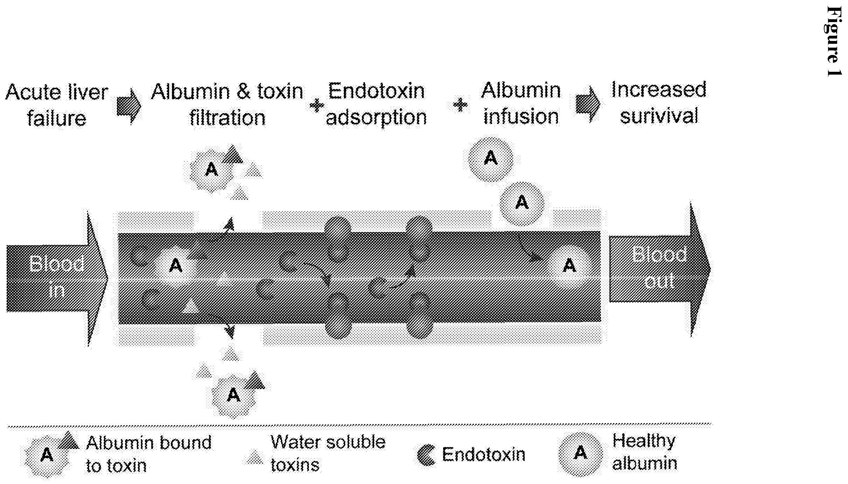

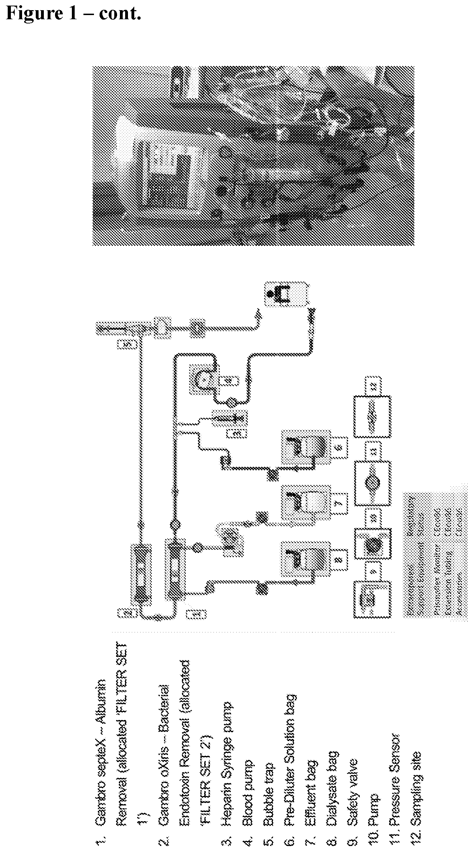

Treatment options for ACLF are currently lacking but a novel strategy is the use of extracorporeal liver assist device, DIALIVE that targets the severe cytokine storm using two novel filters that allows albumin exchange and, removal of damage associated molecular patterns and pathogen associated molecular patterns. DIALIVE has been just finished a randomised and controlled clinical trial in patients with ACLF, shown to be safe effective in reducing the cytokine storm that is a feature of ACLF and improving organ function. The configuration of the DIALIVE device is shown in **Figure 1****.**

### Example 2: A randomised and controlled trial of DIALIVE in patients with ACLF

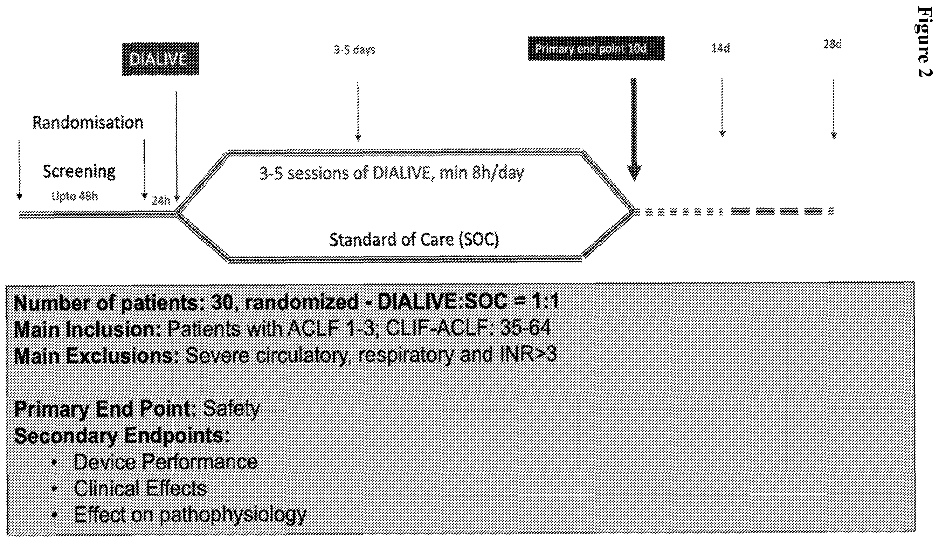

The programme to advance the pre-clinical data on DIALIVE to patients with ACLF was funded by a Euro 6.4M grant from the EU H2020 programme. The study was performed in 11 European hospitals and although full analysis is being performed, preliminary results are presented below. The study design is outlined in **Figure 2****.**

### Brief Results

The study recruited 30 patients with ACLF.

**Safety:** The safety data has been evaluated by the Data Safety Monitoring Board, who have confirmed the safety of the device.

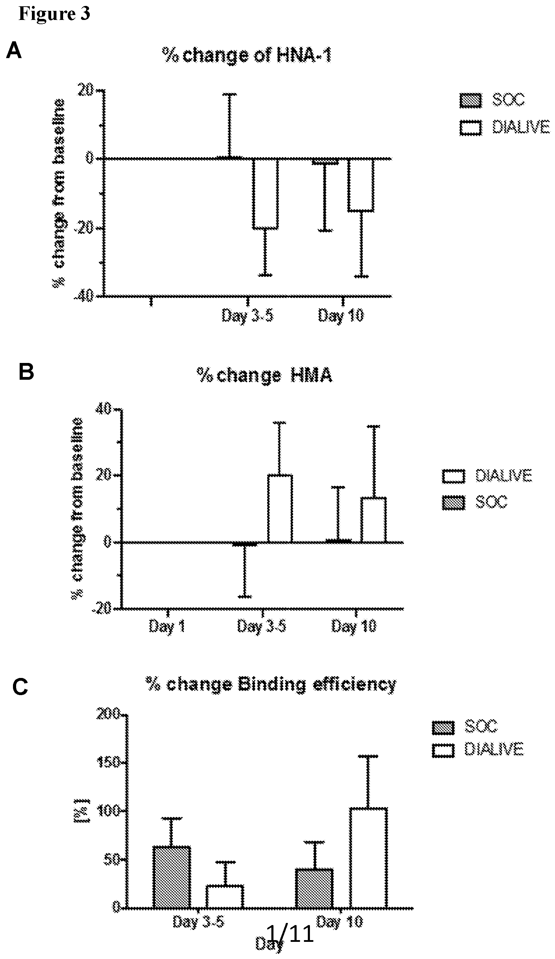

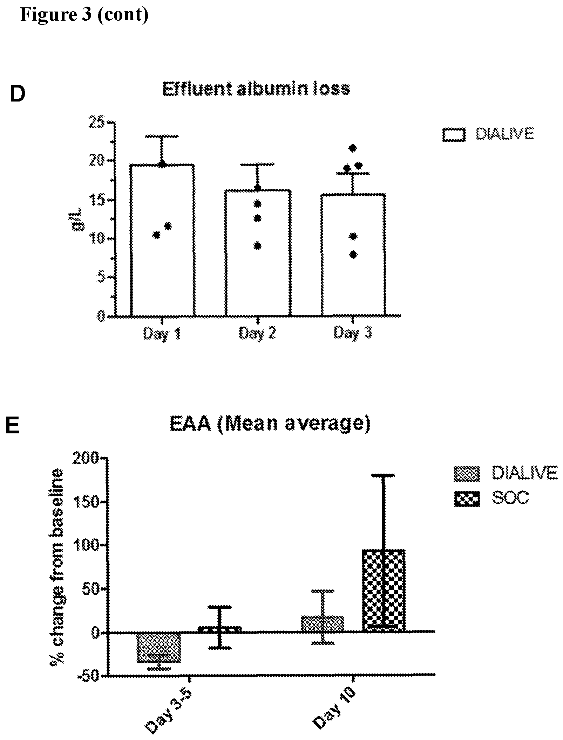

**Performance:** The two main performance indicators for DIALIVE were evidence that it could remove endotoxin, albumin and improve albumin function, see **Figure 3****.**

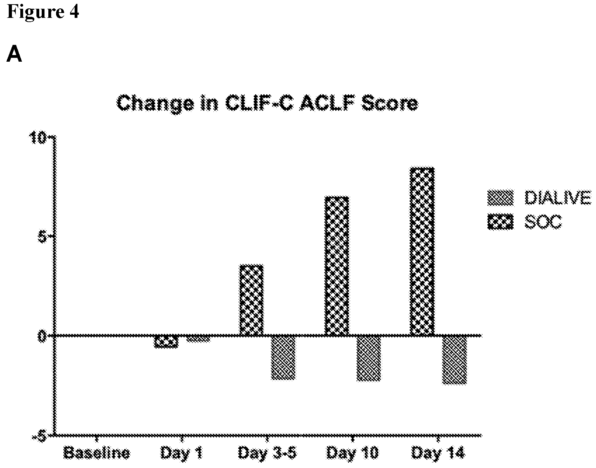

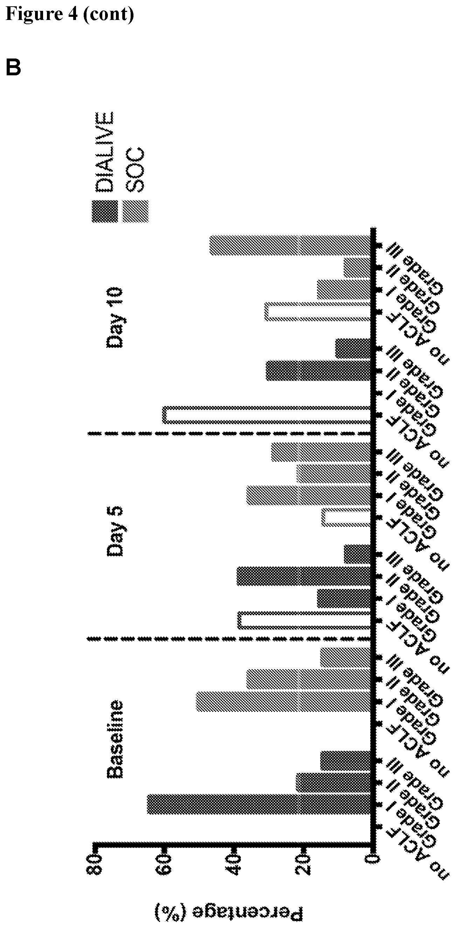

**Effect on Organ Function:** The main efficacy end point that was assessed in this trial was the change in CLIF-ACLF score that is a score from 0-100 and combines the CLIF-organ failure score, age and white cell count. CLIF-organ failure score, which is a modification of the SOFA score, that is widely used in septic patients in the ICU, see **Figure 4****.**

### Effect of DIALIVE on circulating markers of Cell Death and circulating Cytokines:

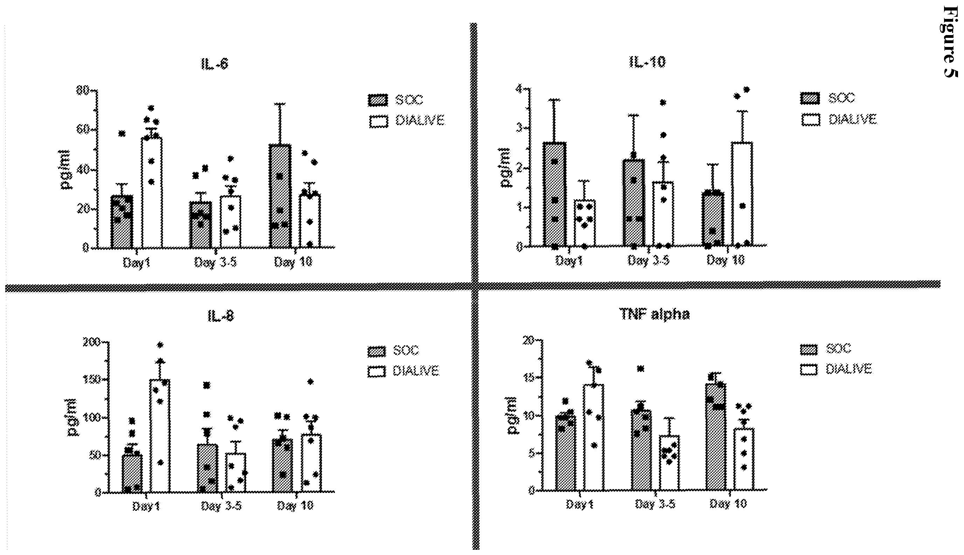

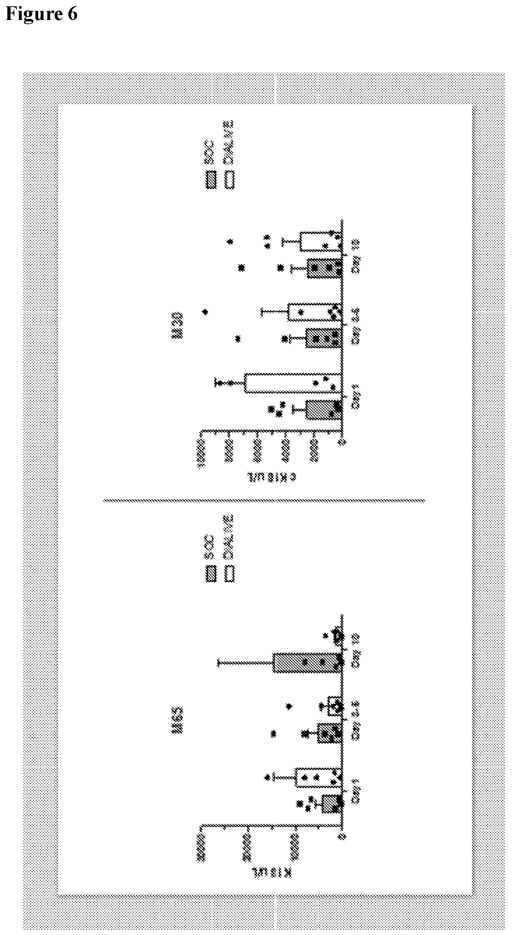

In the randomised patients, markers of cell death and relevant cytokines. The cytokine data from the first 14 patients in this study are presented in **Figure 5****.** **Figure 6** shows a marked reduction in the M30 and M65 fragments of Cytokeratin 18 (markers of cell death) in the patients treated with DIALIVE and standard of care.

### Summary of the effect of DIALIVE in patients with ACLF

The available data show that treatment of ACLF patients with multi-organ failure with DIALIVE is safe and the device performs adequately and to its specifications. In patients treated with DIALIVE, there is a substantial improvement in organ function which is possibly due to a reduction in systemic inflammation, endotoxemia, markers of cell death and favourable modulation of inflammation indicated by changes in cytokinemia.

### Similarities between the cytokine storm in ACLF and severe COVID-19 patients.

The existing data suggests that there are marked similarities in the inflammatory response and the associated immunopathology in patients with ACLF. After reviewing this data, and the emerging data surrounding cytokine storm syndromes, the inventor investigated the immune responses of ACLF patients versus those with severe COVID-19. The inventor analysed the data of Qin et al. 2020 and Claria et al 2016, and in view of that analysis prepared **Figure 7** setting out the striking similarities between the cytokine storms in ACLF and severe COVID-19 patients.

The data show the unexpected finding that removal of endotoxin and exchanging albumin has a profound effect on the severity of cytokinemia and also organ function in patients.

Therefore, devices, such as DIALIVE, that remove endotoxin and exchange albumin can also abrogate cytokine storms, which are characterised by cytokinemia and also organ dysfunction. Such devices abrogate damaging pro-inflammatory cytokines (such as IL6, IL8 and TNFa) while increasing the anti-inflammatory cytokine, IL10.

The data show that there are similarities in the cytokine storm that results in multi-organ failure in ACLF and in patients with severe COVID-19 infection. DIALIVE is safe and addresses the cytokinemia and organ failures that is characteristic of ACLF. Thus, DIALIVE will address the cytokine storm characteristics of severe COVID-19 infection and reduce the mortality of these patients.

### Example 3 - Further data from the DIALIVE clinical trial

The study was based on 32 patients (17 DIALIVE, 15 Standard of care (SOC)) in the Safety set for safety analyses and a subset of 30 patients (15 DIALIVE, 15 SOC) in the Modified Safety set for biomarker and efficacy analyses. Outcome analyses inclusive of safety, biomarkers, and efficacy are limited to 28 days with the exception of 90-day mortality for a subset of patients.

### Endotoxin and albumin measurements

Endotoxin measures supported adequate device performance and efficacy. For EAA (Endotoxin Activity Assay) Average, there were trends towards reduction in the DIALIVE group that was most marked at Day 5. The pre-planned analyses of the Target Value and the Acceptable Value were of primary interest when the CV% was <15%. The optimistic Target Value (40%) was not achieved but the 20% Acceptable Value was achieved; acceptable values were met at Day 3 (pre-treatment) for both groups with best differentiation at Day 5 (pre-treatment) with clinically significant advantage seen for the DIALIVE group (80%) vs SOC (36.4%) at Day 5. There were significant reductions in endotoxin activity measured using the Limulus Amebocyte Lysate (LAL) assay in the DIALIVE patients but no difference in LBP was observed between the groups. The data lend support to the hypothesis that DIALIVE effectively removes circulating endotoxins.

Albumin measures supported evidence of device performance and efficacy. Consistent improvements and advantages were observed for the DIALIVE group across most measures, most of which were statistically significant. The strongest outcomes were observed for HMA%, HMA/HNA Ratio, HNA1%, HNA2%, HNA-2/HMA Ratio, and IMAR where there were clinically and statistically significant improvements for the DIALIVE group at Days 5 and 10 in contrast to minimal changes for SOC. The pre-planned modeling for the absolute change from baseline identified significant DIALIVE advantages; the Treatment (Rx) covariate (across all days) was significant for the 7 measures while the Day 5 outcome was significant for 6 of the measures and the Day 10 outcome was significant for the same 7 measures as the Treatment covariate. All five HMA and HNA based measures were significant for each of Treatment, Day 5, and Day 10.

### Organ function

The analysis of improvement in individual organ function (CLIF Organ Failure components: Brain, Kidney, CVS, Pulmonary, Liver and Coagulation) identified a clear and significant advantage for DIALIVE vs SOC. Improved brain subscore was observed in the DIALIVE group (p<0.001) but not in the SOC group (73.3% vs 20%); this was statistically significant between groups (two-sided Fisher Exact test, p=0.0092). For liver subscore, statistically significant improvement was observed in the DIALIVE group (p=0.045) (53.3% vs 33.3%). There was significant deterioration of lung subscore in the SOC group (p=0.025), which was not observed in the DIALIVE group. Taken together, this resulted in a statistically significant reduction in the CLIF-C organ failure Score at Day 5 and Day 10.

**Table 1 - Improvement in Individual Organ Function**

| **Day/Parameter** | **Category** | **DIALIVE (n=15)** | **SOC (n=15)** |
|---|---|---|---|
| | | **N (%)** | **N (%)** |
| Liver Improvement | Yes | 53.3% (8/15) | 33.3% (5/15) |
| Kidney Improvement | Yes | 20.0% (3/15) | 13.3% (2/15) |
| Brain Improvement | Yes | 73.3% (11/15) | 20.0% (3/15) |
| Coagulation Improvement | Yes | 20.0% (3/15) | 13.3% (2/15) |
| Circulation Improvement | Yes | 0% (0/15) | 6.7% (1/15) |
| Lung Improvement | Yes | 20.0% (3/15) | 13.3% (2/15) |

**Table 2- Organ Function Over the First 10 days**

| Organ | DIALIVE | | | SOC | | |
|---|---|---|---|---|---|---|
| | Baseline (n=15) | Day 5 (n=14) | Day 10 (n=11) | Baseline (n=15) | Day 5 (n=15) | Day 10 (n=13) |
| Liver | **2.9 (0.4)** | **2.6 (0.6)** | ***2.4 (0.9)** | 2.9 (0.5) | 2.8 (0.6) | 2.6 (0.8) |
| Kidney | 1.4 (0.7) | 1.4 (0.8) | 1.3 (0.6) | 1.5 (0.8) | 1.4 (0.7) | 1.2 (0.6) |
| Brain | **1.9 (0.3)** | **1.5 (0.5)** | *****1.4 (0.5)** | 1.5 (0.5) | 1.5 (0.6) | 1.5 (0.8) |
| Coagulation | 1.5 (0.7) | 1.5 (0.5) | 1.4 (0.6) | 1.6 (0.9) | 1.8 (1.0) | 1.9 (0.9) |
| Circulation | 1.1 (0.5) | 1.1 (0.5) | 1.2 (0.6) | 1.1 (0.5) | 1.1 (0.5 | 1.2 (0.6) |
| Respiration | 1.5 (0.8) | 1.5 (0.8) | 1.6 (0.8) | **1.3 (0.6)** | **1.3 (0.6)** | ****1.9 (1.0)** |
| Mean (SD); *p=0.045; **p=0.025; ***p<0.001 | | | | | | |

### Resolution of A CLF (reaching ACLF Grade 0)

There was a 9 day mean advantage for DIALIVE with faster time to resolution of ACLF (ACLF Grade=0) (two-sided t-test p=0.044); significantly more cases reaching ACLF Grade 0 (33.3% vs 66.7%) favouring DIALIVE (two-sided log rank p=0.0357; two-sided t-test p=0.044) and a 2.8x faster time to ACLF Grade 0 which approached statistical significance (two-sided Wald test p=0.059) (**Figure 4B** **and Table 3**). These statistically significant advantages corresponded to a clinically meaningful advantage; during the first 28 days, the DIALIVE patients accrued a total of 245 days with ACLF Grade 0 in contrast to 110 days accrued for the SOC patients.

**Table 3 - ACLF Grade Significance Summary**

| | **DIALIVE (n=15)** | **SOC (n=15)** | **Fisher Exact test/ T-test** | **Log rank test Wald test** |
|---|---|---|---|---|
| % Reaching Goal | 66.7% | 33.3% | 0.1431 (FE) | NA |
| ACLF Grade 0 Days | 245 days | 110 days | NA | NA |
| Mean Days | 16.33 days | 7.33 days | P=0.044 (T) | P=0.0357 (L) |
| % Days ACLF Grade 0 | 54.4% | 24.4% | NA | NA |
| Hazard Ratio | 2.83 (0.96, 8.33) | | NA | P=0.059 (W) |

### Inflammatory markers

For measures of cytokines, the most pronounced effects were observed for TNF-α and IL-8. For TNF-α, there was a borderline treatment effect (two-sided p=.094) and a borderline Day 10 effect (two-sided p=0.053). For IL-8, there was an overall treatment effect (two-sided p=.008) and a day effect (two-sided p=0.019) with significant advantages for DIALIVE at both Day 5 (two-sided p=0.044) and Day 10 (two-sided p=0.006).
For NGAL, there was a significant reduction for the DIALIVE group between Days 5 and 10 (two-sided paired t-test p=0.048) but urinary NGAL did not change. No significant changes were observed in IL1b, IL-6, IL-7, IL-18, CXCL1, CX3CL1, sCD63, CCL5/RANTES and CCL2/MCP.

### Damage Associated Molecular Patterns (DAMPs)

For cytokeratin 18 (M30 cK18 U/L) there was a significant treatment effect overall (two-sided p=0.002), a borderline day effect (two-sided p=0.055), a borderline advantage for DIALIVE at Day 5 (two-sided p=0.081), and a significant advantage for DIALIVE at Day 10 (two-sided p=0.005).

For cytokeratin 18 (M65 K18 U/L) there was a significant treatment effect overall (two-sided p=0.028) and a significant advantage for DIALIVE at Day 10 (two-sided p=0.029).
For the Ratio cK:K18 there was a borderline treatment by day interaction (two-sided p=0.064) and a significant advantage for DIALIVE at Day 5 (two-sided p=0.039).

For Receptor-interacting serine/threonine-protein kinase 3 (RIPK3) there was a borderline treatment effect overall (two-sided p=0.094) and a significant advantage for DIALIVE at Day 5 (two-sided p=0.030).

For each of three measures of DAMPs, there were significant or borderline treatment effects at Days 5 and 10 in support of DIALIVE. The Cytokeratin 18, M30 cK18 U/L marker had a significant treatment effect overall (two-sided p=0.002) a borderline day effect (two-sided p=0.055), a borderline advantage for DIALIVE at Day 5 (two-sided p=0.081), and a significant advantage for DIALIVE at Day 10 (two-sided p=0.005) reflecting a reduction in apoptotic cell death in DIALIVE treated patients. The observed reductions in M65 K18 suggests a reduction in non-apoptotic forms of cell death in the DIALIVE treated patients. For RIPK3, there was a borderline treatment effect overall (two-sided p=0.094) and a significant advantage for DIALIVE at Day 5 (two-sided p=0.030). The trend towards a reduction in RIPK3 suggests a reduction in necroptosis.

### Endothelial markers

Among the four endothelial markers E-Selectin, ICAM-1, VCAM-1 and Coagulation Factor VIII, Coagulation Factor VIII was the most supportive for the beneficial effect of DIALIVE. Coagulation Factor VIII was supportive with a significant treatment effect overall (two-sided p=.009) and a borderline treatment by day interaction (two-sided p=0.059); there was a significant advantage for DIALIVE at Day 5 (two-sided p=0.002). There were no significant trends in E-Selectin, ICAM-1 and VCAM-1.

### Signaling Markers

For the IL-1 β/IL-8 Response Ratio there was an overall significant treatment effect (two-sided p=<0.001) and a nearly significant treatment by day interaction (two-sided p=0.065). There were significant advantages for DIALIVE at both Day 5 (two-sided p<0.001) and Day 10 (two-sided p=0.002).

For the TLR4 Response Ratio there was a significant treatment effect overall (two-sided p=0.003) with significant advantages for DIALIVE at both Day 5 (two-sided p=0.005) and Day 10 (two-sided p=0.03).

Both signalling markers support DIALIVE efficacy. For the IL-1 β/IL-8 Response Ratio, there was an overall significant treatment effect (two-sided p=<0.001) and a significant treatment by day interaction (two-sided p=0.019) with significant advantages for DIALIVE at both Day 5 (two-sided p<0.001) and Day 10 (two-sided p=0.002). Similarly, for the TLR4 Response Ratio, there was a significant treatment effect overall (two-sided p=0.003) with significant advantages for DIALIVE at both Day 5 (two-sided p=.005) and Day 10 (two-sided p=0.03).

### Summary

- DIALIVE efficacy was demonstrated based on percentages and faster time to reach ACLF Grade 0.
   o 66.7% of the DIALIVE group compared with 33.3% of the SOC group reached ACLF 0 by Day 28
   ∘ There was a statistically significant advantage for DIALIVE vs SOC for time to resolution of ACLF Grade (two-sided log rank p=0.0357)
   ∘ There was a mean 9-day advantage for DIALIVE vs SOC for ACLF Grade 0 days (two-sided t-test p=0.044).
- Efficacy was further demonstrated for the improvement in individual organ function, the CLIF-OF score and the MELD score
   ∘ CLIF OF score: There was a significant treatment effect overall (two-sided p=0.025) with significant advantages for DIALIVE at both Day 10 (two-sided p=0.021) and Day 14 (two-sided p=0.001).
   ∘ The brain subscore improved in 73.3% of the DIALIVE group vs 20% of the SOC (p<0.001)
   ∘ The liver subscore improved 53.3% of the DIALIVE group vs 33.3% of the SOC group (p=0.045).
   ∘ The lung subscore deteriorated in the SOC group (p=0.025), which was not observed in the DIALIVE group.
   ∘ MELD and MELD-Na Score were significantly improved in the DIALIVE group compared with the SOC group.
- Multiple biomarkers improved significantly in the DIALIVE treated patients compared with SOC
   o Markers of endothelial function: There was a significant treatment group differences at both Day 5 and Day 10 for Factor VIII and a significant treatment group difference at Day 10 for ADMA.
   ∘ Markers of systemic inflammation: Significant treatment group differences were observed for IL-8 in favour of DIALIVE with concomitant reductions in TNF-α and NGAL.
   ∘ Markers of cell death: Marked treatment differences in favor of DIALIVE were observed for each of Cytokeratin 18 and RIPK3.
   ∘ Cell signaling: Significant treatment group differences were observed at both Day 5 and Day 10 effects for the IL-1 β/IL-8 Response Ratio and for the TLR4 Response Ratio.

### References

Channappanavar R, Perlman S: Pathogenic human coronavirus infections: causes and consequences of cytokine storm and immunopathology. Semin Immunopathol 2017, 39(5):529-539.
Chen T, Wu D, Chen H, Yan W, Yang D, Chen G, Ma K, Xu D, Yu H, Wang H et al: Clinical characteristics of 113 deceased patients with coronavirus disease 2019: retrospective study. BMJ 2020.
Claria, J., et al. (2016), Systemic inflammation in decompensated cirrhosis: Characterization and role in acute-on-chronic liver failure. Hepatology, 64: 1249-1264.
Conti P, Ronconi G, Caraffa A, Gallenga CE, Ross R, Frydas I, Kritas SK: Induction of pro-inflammatory cytokines (IL-1 and IL-6) and lung inflammation by Coronavirus-19 (COVI-19 or SARS-CoV-2): anti-inflammatory strategies. Journal of biological regulators and homeostatic agents 2020, 34(2).
Coronavirus disease 2019 (COVID-19)-Situation Report - 67. WHO report*.*
Falkenhagen et al (Artificial Organs (1996) 20:420)
Harlow and Lane (1988) "Antibodies: A Laboratory Manual", Cold Spring Harbor Laboratory Press, Cold Spring Harbor, NY.
Kohler and Milstein (1975) Nature 256, 495-497).
Leeet al. (2015) "Extracorporeal liver assist device to exchange albumin and remove endotoxin in acute liver failure: Results of a pivotal pre-clinical study". Journal of Hepatology 63(3):634-642.
Maddox et al, J. Exp. Med. 158, 1211-1226, 1993.
Mahase E: Coronavirus covid-19 has killed more people than SARS and MERS combined, despite lower case fatality rate. BMJ 2020, 368:m641.
Moreau, Richard et al. Acute-on-Chronic Liver Failure Is a Distinct Syndrome That Develops in Patients With Acute Decompensation of Cirrhosis. Gastroenterology , Volume 144 , Issue 7 , 1426 -1437.e9
Qin C, Zhou L, Hu Z, Zhang S, Yang S, Tao Y, Xie C, Ma K, Shang K, Wang W et al: Dysregulation of immune response in patients with COVID-19 in Wuhan, China. Clin Infect Dis 2020.
Ruan Q et al. Clinical predictors of mortality due to COVID-19 based on an analysis of data of 150 patients from Wuhan, China. Intensive Care Med. 2020; (published online March 3 2020)
Russell CD, Millar JE, Baillie JK: Clinical evidence does not support corticosteroid treatment for 2019-nCoV lung injury. Lancet 2020, 395(10223):473-475.
Severe Outcomes Among Patients with Coronavirus Disease 2019 (COVID-19) - United States, February 12-March 16, 2020. MMWR Morb Mortal Wkly Rep 2020, 69(12):343-346.
Shang L, Zhao J, Hu Y, Du R, Cao B: On the use of corticosteroids for 2019-nCoV pneumonia. Lancet 2020, 395(10225):683-684.
Staubach et al (Transfusion and Apheresis Science (2003) 29: 93-98)
[The epidemiological characteristics of an outbreak of 2019 novel coronavirus diseases (COVID-19) in China]. Zhonghua Liu Xing Bing Xue Za Zhi 2020, 41(2):145-151.
Yang X, Yu Y, Xu J, Shu H, Xia Ja, Liu H, Wu Y, Zhang L, Yu Z, Fang M et al: Clinical course and outcomes of critically ill patients with SARS-CoV-2 pneumonia in Wuhan, China: a single-centered, retrospective, observational study. The Lancet Respiratory Medicine 2020.
Zhou F, Yu T, Du R, Fan G, Liu Y, Liu Z, Xiang J, Wang Y, Song B, Gu X et al: Clinical course and risk factors for mortality of adult inpatients with COVID-19 in Wuhan, China: a retrospective cohort study. The Lancet 2020, 395(10229):1054-1062.

## Claims

1. Albumin that does not derive from the blood of an individual to be treated for use in a method of treating a cytokine storm syndrome, said method comprising the steps of
(a) removing albumin from the blood of the individual;
(b) reducing the level of endotoxin in the blood of the individual; and
(c) introducing said albumin that does not derive from the individual to be treated into the blood of the individual;
wherein step (a) is carried out by dialysis using means comprising a membrane having a pore size of greater than 50kDa and less than 100 kDa.

2. The albumin for use according to claim 1, wherein:
steps (a) and (b) of the method comprise different means for removing albumin and reducing the level of endotoxin.

3. The albumin for use according to claim 1 or claim 2, wherein steps (a) and (c) are carried out by dialysis.

4. The albumin for use according to any one of claims 1 to 3, wherein: (i) step (b) comprises removing endotoxin from the blood of the individual; or (ii) step (b) comprises administering to the individual a therapeutically effective amount of an agent capable of reducing the level of endotoxin in the blood, optionally wherein the method further comprises the removal of cytokines from the blood of the individual.

5. The albumin for use according to any one of claims 1 to 4, wherein the albumin of step (c) is pharmaceutical grade albumin.

6. The albumin for use according to any one of claims 1 to 5, wherein the method comprises:
(d) removing cytokines from the blood of the individual.

7. The albumin for use according to any one of claims 1 to 6, wherein the method comprises:
(e) removing toxins bound to albumin from the blood of the individual.

8. The albumin for use according to any one of claims 1 to 7, wherein said step (a) comprises the use of a solid support capable of selectively binding albumin.

9. The albumin for use according to any one of claims 1 to 8, wherein said step (b) comprises the use of a solid support capable of selectively binding endotoxin.

10. The albumin for use according to any one of claims 1 to 9, wherein other components removed with the albumin are returned to the blood of the individual.

11. An *in vitro* method of treating blood extracorporeally by selectively removing albumin and endotoxin from the blood, wherein the blood is from an individual having a cytokine storm syndrome, the method comprising:
(a) contacting the blood with a solid support which selectively binds albumin and thereby removing albumin from the blood;
(b) contacting the blood with a solid support which selectively binds endotoxin and thereby removing endotoxin from the blood; and
(c) adding to the blood albumin that does not derive from the same individual as the blood.

12. The method according to claim 11, wherein:
(i) steps (a) and (b) of the method comprise different means for removing albumin and removing endotoxin; and/or
(ii) the solid support of (a) comprises an antibody that specifically binds albumin.

13. The albumin for use, or *in vitro* method, according to any one of the preceding claims, wherein the cytokine storm syndrome is associated with an autoimmune condition, secondary haemophagocytic lymphohistiocytosis (sHLH), a viral infection, severe sepsis, post-surgical multi organ failure, post-liver resection multi organ failure, or post chemotherapy cytokine storm; optionally wherein the viral infection is an influenza infection or a COVID-19 infection.

14. Albumin that does not derive from the blood of an individual to be treated for use in a method of treating an individual with a COVID-19 infection, said method comprising treating said individual's blood by:
(a) selectively removing albumin from the blood of the individual;
(b) selectively removing endotoxin from the blood of the individual; and
(c) supplying albumin that does not derive from the individual to the blood of the individual;
wherein said step (a) comprises dialysis of albumin with means comprising a membrane having a pore size of greater than 50 kDa and less than 100 kDa.

15. Albumin that does not derive from the blood of an individual to be treated for use in a method of treating a COVID-19 patient with multiple organ failure, said method comprising treating said individual's blood by:
(a) selectively removing albumin from the blood of the individual;
(b) selectively removing endotoxin from the blood of the individual; and
(c) supplying albumin that does not derive from the individual to the blood of the individual;
wherein said step (a) comprises use of means for dialysis of albumin, said means comprising a membrane having a pore size of greater than 50 kDa and less than 100 kDa.

## Patentansprüche

1. Albumin, das nicht aus dem Blut eines zu behandelnden Individuums stammt, zur Verwendung in einem Verfahren zur Behandlung eines Zytokinsturmsyndroms, wobei das Verfahren folgende Schritte umfasst:
(a) Entfernen von Albumin aus dem Blut des Individuums;
(b) Reduzieren des Endotoxinspiegels im Blut des Individuums; und
(c) Einbringen des Albumins, das nicht von dem zu behandelnden Individuum stammt, in das Blut des Individuums;
wobei Schritt (a) durch Dialyse unter Verwendung eines Mittels durchgeführt wird, das eine Membran mit einer Porengröße von mehr als 50 kDa und weniger als 100 kDa umfasst.

2. Albumin zur Verwendung nach Anspruch 1, wobei:
die Schritte (a) und (b) des Verfahrens unterschiedliche Mittel zur Entfernung von Albumin und zur Verringerung des Endotoxinspiegels umfassen.

3. Albumin zur Verwendung nach Anspruch 1 oder Anspruch 2, wobei die Schritte (a) und (c) durch Dialyse durchgeführt werden.

4. Albumin zur Verwendung nach einem der Ansprüche 1 bis 3, wobei: (i) Schritt (b) die Entfernung von Endotoxin aus dem Blut des Individuums umfasst; oder (ii) Schritt (b) die Verabreichung einer therapeutisch wirksamen Menge eines Wirkstoffs an das Individuum umfasst, der in der Lage ist, den Endotoxinspiegel im Blut zu reduzieren, optional wobei das Verfahren ferner die Entfernung von Zytokinen aus dem Blut des Individuums umfasst.

5. Albumin zur Verwendung nach einem der Ansprüche 1 bis 4, wobei das Albumin von Schritt (c) pharmazeutisches Albumin ist.

6. Albumin zur Verwendung nach einem der Ansprüche 1 bis 5, wobei das Verfahren umfasst:
(d) Entfernen von Zytokinen aus dem Blut des Individuums.

7. Albumin zur Verwendung nach einem der Ansprüche 1 bis 6, wobei das Verfahren umfasst:
(e) Entfernen von an Albumin gebundenen Toxinen aus dem Blut des Individuums.

8. Albumin zur Verwendung nach einem der Ansprüche 1 bis 7, wobei der Schritt (a) die Verwendung eines festen Trägers umfasst, der in der Lage ist, selektiv Albumin zu binden.

9. Albumin zur Verwendung nach einem der Ansprüche 1 bis 8, wobei der Schritt (b) die Verwendung eines festen Trägers umfasst, der in der Lage ist, selektiv Endotoxin zu binden.

10. Albumin zur Verwendung nach einem der Ansprüche 1 bis 9, wobei andere mit dem Albumin entfernte Bestandteile in das Blut des Individuums zurückgeführt werden.

11. In-vitro-Verfahren zur extrakorporalen Behandlung von Blut durch selektive Entfernung von Albumin und Endotoxin aus dem Blut, wobei das Blut von einem Individuum stammt, das ein Zytokinsturmsyndrom hat, wobei das Verfahren umfasst:
(a) Inkontaktbringen des Blutes mit einem festen Träger, der selektiv Albumin bindet und dadurch Albumin aus dem Blut entfernt;
(b) Inkontaktbringen des Blutes mit einem festen Träger, der selektiv Endotoxin bindet und dadurch Endotoxin aus dem Blut entfernt; und
(c) Zugeben von Albumin zu dem Blut, das nicht von demselben Individuum wie das Blut stammt.

12. Verfahren nach Anspruch 11, wobei:
(i) die Schritte (a) und (b) des Verfahrens unterschiedliche Mittel zur Entfernung von Albumin und zur Entfernung von Endotoxin umfassen; und/oder
(ii) der feste Träger von (a) einen Antikörper umfasst, der spezifisch Albumin bindet.

13. Albumin zur Verwendung, oder In-vitro-Verfahren, nach einem der vorhergehenden Ansprüche, wobei das Zytokinsturmsyndrom einer Autoimmunerkrankung, einer sekundären hämophagozytischen Lymphohistiozytose (sHLH), einer viralen Infektion, einer schweren Sepsis, einem postoperativen Multiorganversagen, einem Multiorganversagen nach einer Leberresektion oder einem Zytokinsturm nach einer Chemotherapie zugeordnet ist; optional wobei die virale Infektion eine Influenza-Infektion oder eine COVID-19-Infektion ist.

14. Albumin, das nicht aus dem Blut eines zu behandelnden Individuums stammt, zur Verwendung in einem Verfahren zur Behandlung eines Individuums mit einer COVID-19-Infektion, wobei das Verfahren die Behandlung des Blutes des Individuums durch Folgendes umfasst:
(a) selektives Entfernen von Albumin aus dem Blut des Individuums;
(b) selektives Entfernen von Endotoxin aus dem Blut des Individuums; und
(c) Zuführen von Albumin, das nicht von dem Individuum stammt, zu dem Blut des Individuums;
m wobei der Schritt (a) die Dialyse von Albumin mit Mitteln umfasst, die eine Membran mit einer Porengröße von mehr als 50 kDa und weniger als 100 kDa aufweisen.

15. Albumin, das nicht aus dem Blut eines zu behandelnden Individuums stammt, zur Verwendung in einem Verfahren zur Behandlung eines COVID-19-Patienten mit multiplem Organversagen, wobei das Verfahren die Behandlung des Blutes des Individuums durch Folgendes umfasst:
(a) selektives Entfernen von Albumin aus dem Blut des Individuums;
(b) selektives Entfernen von Endotoxin aus dem Blut des Individuums; und
(c) Zuführen von Albumin, das nicht von dem Individuum stammt, zu dem Blut des Individuums;
wobei der Schritt (a) die Verwendung eines Mittels zur Dialyse von Albumin umfasst, wobei das Mittel eine Membran mit einer Porengröße von mehr als 50 kDa und weniger als 100 kDa umfasst.

## Revendications

1. Albumine qui ne provient pas du sang d'un individu devant être traité, pour utilisation dans une méthode de traitement d'un syndrome d'orage cytokinique, ladite méthode comprenant les étapes suivantes :
(a) éliminer de l'albumine du sang de l'individu ;
(b) réduire le niveau d'endotoxine dans le sang de l'individu ; et
(c) introduire ladite albumine, qui ne provient pas de l'individu devant être traité, dans le sang de l'individu ;
dans laquelle l'étape (a) est effectuée par dialyse en utilisant des moyens comprenant une membrane ayant une taille des pores supérieure à 50 kDa et inférieure à 100 kDa.

2. Albumine pour utilisation selon la revendication 1, dans laquelle :
les étapes (a) et (b) de la méthode comprennent des moyens différents pour éliminer de l'albumine et réduire le niveau d'endotoxine.

3. Albumine pour utilisation selon la revendication 1 ou la revendication 2, dans laquelle les étapes (a) et (c) sont effectuées par dialyse.

4. Albumine pour utilisation selon l'une quelconque des revendications 1 à 3, dans laquelle : (i) l'étape (b) comprend le fait d'éliminer de l'endotoxine du sang de l'individu ; ou (ii) l'étape (b) comprend le fait d'administrer, à l'individu, une quantité thérapeutiquement efficace d'un agent capable de réduire le niveau d'endotoxine dans le sang, facultativement dans laquelle la méthode comprend en outre l'élimination de cytokines du sang de l'individu.

5. Albumine pour utilisation selon l'une quelconque des revendications 1 à 4, dans laquelle l'albumine de l'étape (c) est de l'albumine de qualité pharmaceutique.

6. Albumine pour utilisation selon l'une quelconque des revendications 1 à 5, dans laquelle la méthode comprend :
(d) le fait d'éliminer des cytokines du sang de l'individu.

7. Albumine pour utilisation selon l'une quelconque des revendications 1 à 6, dans laquelle la méthode comprend :
(e) le fait d'éliminer des toxines, liées à l'albumine, du sang de l'individu.

8. Albumine pour utilisation selon l'une quelconque des revendications 1 à 7, dans laquelle ladite étape (a) comprend l'utilisation d'un support solide capable de se lier sélectivement à l'albumine.

9. Albumine pour utilisation selon l'une quelconque des revendications 1 à 8, dans laquelle ladite étape (b) comprend l'utilisation d'un support solide capable de se lier sélectivement à l'endotoxine.

10. Albumine pour utilisation selon l'une quelconque des revendications 1 à 9, dans laquelle d'autres composants éliminés avec l'albumine sont restitués au sang de l'individu.

11. Méthode *in vitro* de traitement extracorporel de sang en éliminant sélectivement de l'albumine et de l'endotoxine du sang, dans laquelle le sang provient d'un individu présentant un syndrome d'orage cytokinique, la méthode comprenant les faits :
(a) de mettre le sang en contact avec un support solide qui se lie sélectivement à l'albumine et ainsi d'éliminer l'albumine du sang ;
(b) de mettre en contact le sang avec un support solide qui se lie sélectivement à l'endotoxine et ainsi d'éliminer l'endotoxine du sang ; et
(c) d'ajouter de l'albumine sanguine qui ne provient pas du même individu que le sang.

12. Procédé selon la revendication 11, dans laquelle :
(i) les étapes (a) et (b) de la méthode comprennent des moyens différents pour éliminer l'albumine et éliminer l'endotoxine ; et/ou
(ii) le support solide de (a) comprend un anticorps qui se lie spécifiquement à l'albumine.

13. Albumine pour utilisation, ou méthode *in vitro,* selon l'une quelconque des revendications précédentes, dans laquelle le syndrome d'orage cytokinique est associé à une condition auto-immunitaire, une lymphohistiocytose hémophagocytaire secondaire (sHLH), une infection virale, un sepsis sévère, une défaillance multiviscérale post-chirurgicale, une défaillance multiviscérale post-résection hépatique, ou un orage cytokinique post-chimiothérapie ; facultativement dans laquelle l'infection virale est une infection grippale, ou une infection par COVID-19.

14. Albumine qui ne provient pas du sang d'un individu devant être traité, pour utilisation dans une méthode de traitement d'un individu avec une infection par COVID-19, ladite méthode comprenant le traitement du sang dudit individu en :
(a) éliminant sélectivement de l'albumine du sang de l'individu ;
(b) éliminant sélectivement de l'endotoxine du sang de l'individu ; et
(c) fournissant de l'albumine, qui ne provient pas de l'individu, au sang de l'individu ;
dans laquelle ladite étape (a) comprend la dialyse d'albumine avec des moyens comprenant une membrane ayant une taille des pores supérieure à 50 kDa et inférieure à 100 kDa.

15. Albumine qui ne provient pas du sang d'un individu devant être traité, pour utilisation dans une méthode de traitement d'un patient atteint de COVID-19 avec défaillance multiviscérale, ladite méthode comprenant le traitement du sang dudit individu en :
(a) éliminant sélectivement de l'albumine du sang de l'individu ;
(b) éliminant sélectivement de l'endotoxine du sang de l'individu ; et
(c) fournissant de l'albumine, qui ne provient pas de l'individu, au sang de l'individu ;
dans laquelle ladite étape (a) comprend l'utilisation de moyens pour dialyse d'albumine, lesdits moyens comprenant une membrane ayant une taille des pores supérieure à 50 kDa et inférieure à 100 kDa.
